# EUROPÄISCHE PATENTSCHRIFT

(11) **EP 2 095 800 B1**
(45) Veröffentlichungstag und Bekanntmachung des Hinweises auf die Patenterteilung: **17.08.2011**
(21) Anmeldenummer: 08003865.6
(22) Anmeldetag: 01.03.2008
(51) Int. Cl.: A61F 13/06

(54) **Druckverband**
Compression bandage
Bande compressive

(43) Veröffentlichungstag der Anmeldung: 02.09.2009
(73) Patentinhaber: Karl Werkmeister, Medizinische Leibbinden Inh. Hans-Jürgen Germerodt, 37281 Wanfried (DE)
(72) Erfinder: Germerodt, Hans-Jürgen, 37281 Wanfried (DE)
(74) Vertreter: Walther, Robert

(56) Entgegenhaltungen:
- GB-A- 193 096
- GB-A- 981 538
- US-A- 4 957 105

## Beschreibung

Die Erfindung betrifft einen Druckverband, umfassend einen Bauchgurt sowie einen ersten Beingurt, wobei der Beingurt über die Leistengegend geführt ist, wobei der Beingurt an zumindest einem Ende lösbar mit dem Bauchgurt verbindbar ist, mit einem weiteren Beingurt, der ebenfalls über die Leistengegend führbar ist, wobei der weitere Beingurt an beiden Enden lösbar mit dem Bauchgurt verbindbar ist, wobei der weitere Beingurt zu beiden Enden jeweils durch einen Klettverschluss mit dem Bauchgurt verbindbar ist.

Ein Druckverband mit einem Beingurt ist im Handel erhältlich.

Im Bereich der Leiste wird sich am Oberschenkel zu Herzuntersuchungen häufig Zugang zum Herzen durch die Vene oder Arterie verschafft. Das heißt, die Vene oder die Arterie wird punktiert. Nach Abschluss der Herzuntersuchung muss dafür gesorgt werden, dass sich die Vene oder die Arterie verschließt. Hierzu wird ein Kompressorium auf die Punktion gelegt, und mittels des zuvor genannten Druckverbandes dieses Kompressorium gegen die Punktion in der Arterie oder Vene gepresst. Nach einiger Zeit verschließt sich dann die Vene oder die Arterie wieder.

Nun gibt es sogenannte elektrophysiologische Untersuchungen, bei der die Vene oder Arterie an beiden Oberschenkeln punktiert wird. Eine solche elektrophysiologische Untersuchung ist z. B. erforderlich, um Herz/Rhythmusstörungen feststellen zu können. Um nunmehr Punktionsstellen versorgen zu können, wurde zum einen der bereits bekannte Druckverband angelegt, wobei die andere Punktionsstelle in einem Kompressorium und einer gesonderten elastischen Binde versorgt wurde. Nachteilig hierbei ist, dass diese Binde leicht verrutscht, insbesondere dann, wenn sich der Patient bewegt. Das heißt, Voraussetzung hierfür ist, dass der Patient während der Phase, in der sich die Punktion verschließt, liegen bleibt. Eine andere Möglichkeit bestünde darin, zwei der bekannten Druckverbände anzulegen, dies allerdings mit der Folge, dass im Bauchbereich der Bauchgurt insofern doppelt verläuft. Dies ist aufgrund der Einschnürung des Bauches für den Patienten äußerst unangenehm.

Um nunmehr beide Punktionen an beiden Oberschenkeln im Bereich der Leiste zu gleicher Zeit verschließen zu können, ist aus der US 4,975,105 bekannt, einen Druckverband mit zwei Beingurten vorzusehen.

Gegenstand der Erfindung ist nun, dass der Klettverschluss doppelseitig wirkend ausgebildet ist, so dass der Klettverschluss als Pad an beliebiger Stelle auf dem Gurtangeordnet werden kann, d. h. der Gurt entsprechend der vorgesehenen Länge abgeschnitten wird, das Pad auf dem Gurt aufgebracht wird und dann der Gurt mit Pad mit dem Bauchgurt verbunden wird. Grundsätzlich gilt, dass die Verwendung von Klettverschlusspads an jeder Stelle, also sowohl zur Fixierung des ersten Beingurtes als auch des weiteren zweiten Beingurtes und auch des Bauchgurtes möglich ist.

In Bezug auf den Bauchgurt ist ebenfalls vorgesehen, dass zur Anpassung des Bauchgurtes an den Bauchumfang der Bauchgurt einen Klettverschluss aufweist. Für das Material insbesondere der Beingurte ist ein elastisches Material vorgesehen, um entsprechenden Druck aufgrund der elastischen Eigenschaften auf das Kompressorium auszuüben, das auf der Punktion der Vene oder Arterie aufliegt. Aber auch der Bauchgurt kann aus elastisch nachgiebigem Material gefertigt sein.

Anhand der Zeichnungen wird die Erfindung nachstehend beispielhaft näher erläutert.
- Figur 1: zeigt den Druckverband in einer schematischen, perspektivischen Darstellung;
- Figuren 2a-2d: zeigen das Verfahren zum Anlegen des Druckverbandes.

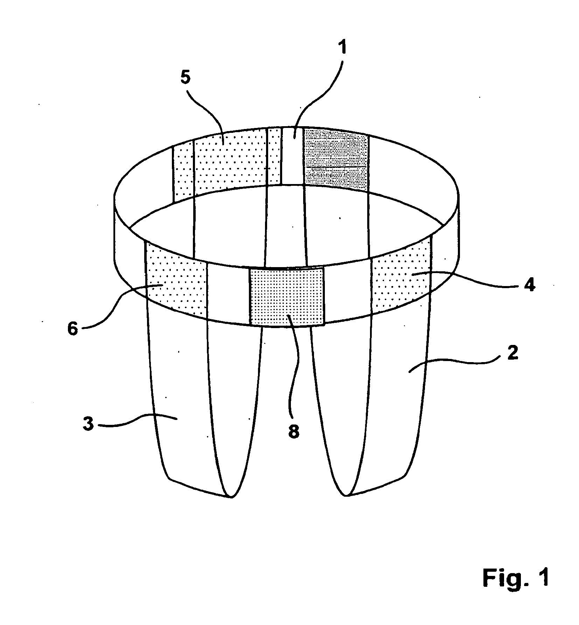

Wie sich aus der Darstellung gemäß Figur 1 ergibt, ist der Bauchgurt insgesamt mit 1 bezeichnet. Hierbei ist ein erster Beingurt 2 und ein weiterer zweiter Beingurt 3 vorgesehen. Der Beingurt 2 ist im hinteren Bereich des Bauchgurtes, also im Rückenbereich des Patienten mit dem Bauchgurt 1 vernäht. Im vorderen Bereich, d. h. im Bauchbereich, befindet sich auf dem Bauchgurt ein Klettverschluss, der mit 4 bezeichnet ist, und der der Fixierung des ersten Beingurtes 2 dient. Für die Fixierung des weiteren zweiten Beingurtes 3 ist wiederum im Bereich des Rückens des Patienten am Bauchgurt 1 ein weiterer Klettverschluss 5 vorgesehen, der sich in etwa in der doppelten Breite des Beingurtes über die Länge des Bauchgurtes erstreckt, um genügend Raum zur Anpassung an die Körperumfänge zu haben. Wiederum im Bauchbereich weist der Bauchgurt einen Klettverschluss 6 zur Fixierung des Beingurtes 3 an dem Bauchgurt 2 im Bauchbereich auf. Der Bauchgurt selbst besitzt zum Verschluss ebenfalls einen Klettverschluss 8. Mit Hilfe der Klettverschlüsse ist es nun möglich, sowohl den Bauchgurt als auch die Beingurte an den jeweiligen Körperumfang anzupassen.

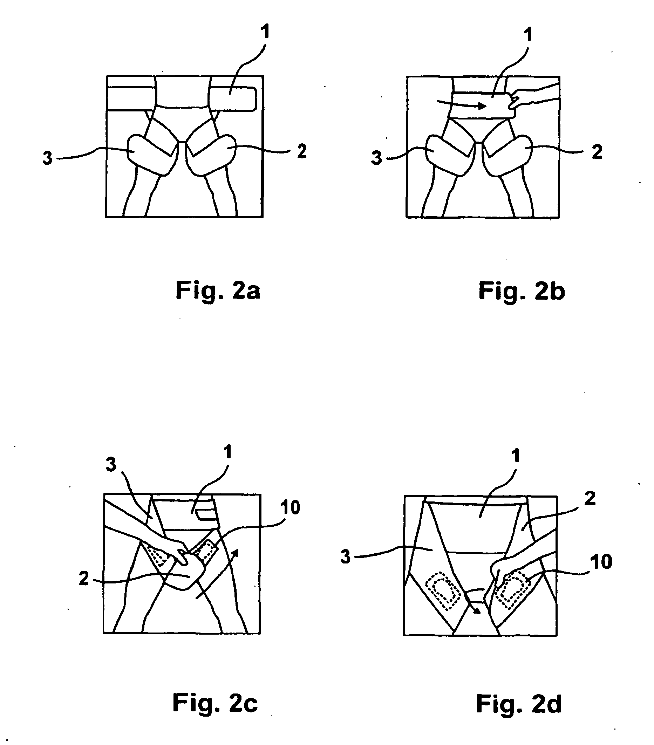

Aus den Figuren 2a bis 2d erschließt sich der Vorgang der Anbringung des Druckverbandes am Patienten. Erkennbar befindet sich hierbei unter dem jeweiligen Beingurt ein Kompressorium 10, beispielsweise in Form eines Stückes Mull, auf das der Beingurt aufgrund der elastischen Ausbildung des Druckverbandes insgesamt Druck ausübt und hierdurch die Punktion verschließt.

## Patentansprüche

1. Druckverband, umfassend einen Bauchgurt (1), sowie einen ersten
Beingurt (2), wobei der Beingurt (2) über die Leistengegend geführt ist, wobei der Beingurt (2) an zumindest einem Ende lösbar mit dem
Bauchgurt (1) verbindbar ist, mit einem weiteren Beingurt (3), der ebenfalls über die Leistengegend führbar ist, wobei der weitere Beingurt (3) an beiden Enden lösbar mit dem Bauchgurt (1) verbindbar ist, wobei der weitere Beingurt (3) zu beiden Enden jeweils durch einen Klettverschluss (5, 6) mit dem Bauchgurt verbindbar ist
**gekennzeichnet durch**
dass der Klettverschluss (4, 5, 6) doppelseitig wirkend ausgebildet ist, so dass der Klettverschluss als Pad an beliebiger Stelle auf dem Gurt angeordnet werden kann.

2. Druckverband nach Anspruch 1,
**dadurch gekennzeichnet,**
**dass** zur Anpassung des Bauchgurtes (1) an den Bauchumfang der Bauchgurt (1) einen Klettverschluss (8) aufweist.

3. Druckverband nach einem der voranstehenden Ansprüche,
**dadurch gekennzeichnet,**
**dass** der erste Beingurt (2) endseitig mittels eines Klettverschlusses (4) mit dem Bauchgurt (1) verbindbar ist.

4. Druckverband nach einem der voranstehenden Ansprüche, **dadurch gekennzeichnet,**
**dass** die Beingurte (2, 3) aus elastisch nachgiebigem Material ausgebildet sind.

## Claims

1. Pressure bandage, comprising a stomach belt (1) and a first leg belt (2), wherein the leg belt (2) is led over the groin, wherein the leg belt (2) is detachably connectible at at least one end with the stomach belt (1), and comprising a further leg belt (3) which can be similarly led over the groin, wherein the further leg belt (3) is detachably connectible at both ends with the stomach belt (1) and wherein the further leg belt (3) is connectible at each of the two ends thereof with the stomach belt by way of a respective hook-and-burr fastener (5, 6), **characterised in that** the hook-and-burr fastener (4, 5, 6) is constructed to have double-sided action so that the hook-and-burr fastener can be arranged as a pad at any position on the belt.

2. Pressure bandage according to claim 1, **characterised in that** the stomach belt (1) has a hook-and-burr fastener (8) for adaptation of the stomach belt (1) to the stomach circumference.

3. Pressure bandage according to one of the preceding claims, **characterised in that** the first leg belt (2) is connectible at the end with the stomach belt (1) by means of a hook-and-burr fastener (4).

4. Pressure bandage according to any one of the preceding claims, **characterised in that** the leg belts (2, 3) are made of elastically flexible material.

## Revendications

1. Bandage compressif comprenant une sangle ventrale (1) ainsi qu'une première sangle de jambe (2), dans lequel la sangle de jambe (2) est passée sur la région de l'aine, dans lequel la sangle de jambe (2) peut être reliée à la sangle ventrale (1) de façon amovible à au moins une extrémité, et comprend une autre sangle de jambe (3) qui peut être passée elle aussi sur la région de l'aine, cette autre sangle de jambe (3) pouvant être reliée à la sangle ventrale (1) de façon amovible à ses deux extrémités, et dans lequel cette autre sangle de jambe (3) peut être reliée à la sangle ventrale à ses deux extrémités, à chaque fois par une fermeture velcro (5, 6),
**caractérisé en ce que**
la fermeture velcro (4, 5, 6) est à action double face, de sorte que la fermeture velcro peut être disposée sous la forme d'un coussinet en n'importe quel endroit sur la sangle.

2. Bandage compressif selon la revendication 1,
**caractérisé en ce que,**
pour l'adaptation de la sangle ventrale (1) au tour de ventre, la sangle ventrale (1) comporte une fermeture velcro (8).

3. Bandage compressif selon l'une des revendications précédentes, **caractérisé en ce que**
la première sangle de jambe (2) peut être reliée en bout à la sangle ventrale (1) au moyen d'une fermeture velcro (4).

4. Bandage compressif selon l'une des revendications précédentes, **caractérisé en ce que**
les sangles de jambe (2, 3) sont réalisées en une matière capable de céder élastiquement.
